Europäisches Patentamt

European Patent Office

Office européen des brevets

(19)

(11) Veröffentlichungsnummer: **0 151 987 B1**

## (12) EUROPÄISCHE PATENTSCHRIFT

(45) Veröffentlichungstag der Patentschrift: **29.05.91**

(51) Int. Cl.5: **A61K 31/355, A61K 9/06**

(21) Anmeldenummer: **85100749.2**

(22) Anmeldetag: **25.01.85**

Teilanmeldung 89120921.5 eingereicht am 25/01/85.

Die Akte enthält technische Angaben, die nach dem Eingang der Anmeldung eingereicht wurden und die nicht in dieser Patentschrift enthalten sind.

(54) **Mittel zur Behandlung von Erkrankungen der Venen und des Analbereichs.**

(30) Priorität: 28.01.84 DE 3402930
15.02.84 DE 3405240
27.02.84 DE 3407024
27.02.84 DE 3407026
07.03.84 DE 3408260
24.04.84 DE 3415250
02.05.84 DE 3416162
17.07.84 DE 3427193
07.09.84 DE 3432881

(43) Veröffentlichungstag der Anmeldung:
**21.08.85 Patentblatt 85/34**

(45) Bekanntmachung des Hinweises auf die Patenterteilung:
**29.05.91 Patentblatt 91/22**

(84) Benannte Vertragsstaaten:
**AT BE CH DE FR GB IT LI LU NL SE**

(56) Entgegenhaltungen:
**DE-A- 2 425 222**
**GB-A- 1 135 709**
**US-A- 3 932 634**
**US-A- 4 169 143**

(73) Patentinhaber: **Ismail, Roshdy, Dr.**
**Siebengebirgs-Apotheke Siebengebirgsallee 2**
**W-5000 Köln 41 (Klettenberg)(DE)**

(72) Erfinder: **Ismail, Roshdy, Dr.**
**Siebengebirgs-Apotheke Siebengebirgsallee 2**
**W-5000 Köln 41 (Klettenberg)(DE)**

(74) Vertreter: **Werner, Hans-Karsten, Dr. et al**
**Deichmannhaus am Hauptbahnhof**
**W-5000 Köln 1(DE)**

DICTIONNAIRE VIDAL, 1984, Cahier complementaire, OVP, Paris, FR

ROTE LISTE, 1983, Editio Cantor,
Aulendorf/Württ., DE

B. HELWIG: "Moderne Arzneimittel", 5. Auflage 1980, Wissenschaftliche Verlagsgesellschaft, Stuttgart, DE;

ROTE LISTE 1981 19005 FF (HEPARIN)

ROTE LISTE 1981 19005 FF (HEPARIN)

MERCK INDEX 10TH EDITION 4575
"HEXACHLOROPHENE"

Rote Liste 1981 52215 Aa "salus" R Herz
Schutz Kapseln

Rote Liste 1983 83124 "Eusovit R 300" 83125
"EVicotrat R Kapseln forte 52256
"Fegacoren" R "N" pro inj.

## Beschreibung

Die vorliegende Erfindung betrifft Mittel zur Behandlung von Erkrankungen des Venen- und Analbereichs unter Verwendung von Vitamin E.

Vitamin E ist bekannt als Antioxidans und Schutzvitamin für Phospholipide der Zellmembran. Es hält die Permeabilität und Stabilität der Zellmembran aufrecht; Lucy, Ann. N.Y. Academy of Science 203 1972, S. 4. Es ist weiterhin bekannt, daß Vitamin E eine membran-abdichtende Wirkung besitzt; F. Mittelbach und G. Bodechtel, Münchner Medizinische Wochenschrift 110 (1968) 36, S. 1988-1993. Bei Erythrocyten, den einfachsten Zellen des menschlichen Körpers wurde festgestellt, daß Vitamin E eine Schutzwirkung für die Zellmembran darstellt. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685-704 (1968) und Phelps DL Pediatrics 63 (6) S. 933-935 (1979). Aus diesen Literaturstellen geht hervor, daß bei Gabe von 200 bis 800 mg Vitamin E oral für einen Zeitraum von 1 bis 4 Tagen, die Hämolyse der Erythrocyten significant verbessert wird im Vergleich zu Patienten mit Vitamin-E-Mangel.

Weiterhin ist bekannt, daß 750 mg Vitamin E täglich in einem Zeitraum von 3 bis 6 Monaten erfolgreich bei Thalassamie-Patienten eingesetzt wurde, wobei eine Normalisierung der Hämolyse der Erythrocyten beobachtet wurde; vgl. Kahane I. ISR. J. med. 12 (1), S. 11-15 (1976).

Es ist weiterhin bekannt, daß Benzaronsalbe und Tabletten zur Behandlung von Beinvenenerkrankungen und Krampfadern sowie damit verbundenen Störungen verwendet werden. Ein Nachteil dieses Wirkstoffes ist, daß bei Lichteinwirkungen Hautreaktionen, Photosensibilität sowie Allergien auftreten.

In B. Helwig, Moderne Arzneimittel, 5. Auflage, S. 1446 bis 1447 (1980) ist ausgeführt, daß der Wirkungsmechanismus des Vitamin E nur zum Teil geklärt ist. Insbesondere greift danach Vitamin E in den Umsatz und die Biosynthese von Kohlenhydraten, Eiweißkörpern, Kreatin- und Nukleinsäuren ein. Die Beschleunigung der Gewebereinigung bzw. -entgiftung durch hohe Vitamin-E-Dosen wurde ebenfalls beobachtet. Bestimmte Vitamin-E-Konzentrationen und Kombinationen von Vitamin E mit anderen Wirkstoffen wurden jedoch nicht untersucht.

Aus der DE-A 2 425 222 sind Vitamin E sowie Vincamin und gegebenenfalls auch Troxerutin enthaltende Mittel in Kapselform oder als Suppositorien bekannt, worin die Troxerutin-Komponente als "Schutzfaktor für die Venen- und Kapillarwand" bezeichnet wird. Die US-A 4,169,143 offenbart für die topische Anwendung wie auch als Suppositorien geeignete Zusammensetzungen, die Vitamin E in einer Menge von etwa wenigstens 200 I. E. und Benzoin enthalten. Beide Druckschriften nennen jedoch transdermal resorbierbare Wirkstoff-Kombinationen mit Vitamin E und einem gefäßerweiternden und/oder durchblutungsfördernden Wirkstoff nicht.

Aus W 083/01898 ist eine pharmazeutische Wirkstoffkombination bekannt, die Vitamin A, Vitamin E, Mandelöl, Sesamöl und Olivenöl enthält. Der Nachteil einer derartigen Zusammensetzung liegt darin, daß Vitamin E schlecht von der Haut aufgenommen wird. Außerdem hat Vitamin E mit Sicherheit keine Wirkung, da es lediglich in niedrigen Konzentrationen zugesetzt wird.

Es wurde erfindungsgemäß überraschenderweise gefunden, daß Vitamin-E-Kombinationen mit gefäßerweiternden und/oder durchblutungsfördernden Mitteln zur Behandlung von Erkrankungen des Venen- und Analbereichs geeignet sind. Dieser neue Indikationsbereich war aufgrund des bisherigen Wissenstandes nicht vorherzusehen und eröffnet ein neues breites Anwendungsfeld für Vitamin E.

Die Erfindung betrifft Mittel zur Behandlung von Erkrankungen der Venen und des Analbereichs im Wege der transdermalen Resorption der Wirkstoffe, gekennzeichnet durch einen Gehalt an Vitamin E im Bereich von 5 bis 20 Gew.-%, an einem oder mehreren gefäßerweiternden und/oder durchblutungsfördernden Mitteln aus der Gruppe Heparin, o-(β-Hydroxyethyl-)Rutoside, Trimethylolrutosid, Extractum Hippocastani, Extractum Arnicae, Extractum Calendulae, Ol. Juniperi, Ol. Pini Pumilionis, Ol. Eucalypti, Ol. Rosmarinae, Tinct. Camphorae, Campher, Salicylsäure und Salicylate, β-Hydroxyethylsalicylat, Nicotinsäure und Nicotinate, Xantinolnicotinat, Inositolnicotinat, Cinnarizin, Bencyclanhydrogenfumarat, Dihydroergotoxinmethansulfonat, Dihydroergocorninmethansulfonat, Dihydroergocristinmethansulfonat, Pentoxifyllin, β-Pyridylcarbinol, Bamethansulfat, Ginkgoflavonglykoside, Flunarizin und dessen Hydrochlorid und Buflomedil, sowie gegebenenfalls an weiteren Wirkstoffen und üblichen Träger- und Hilfsstoffen.

Unter Venenerkrankungen versteht man variköses postthrombotisches Syndrom. Typische Indikationen sind somit Krampfadern mit den Anzeichen Schmerzen, nächtliche Beinkrämpfe und Schwellung. Auch chronische venöse Insuffizienz kann erfindungsgemäß schneller gelindert werden und bringt auf lange Sicht wesentliche Dauerverbesserung.

Es wurde überraschenderweise gefunden, daß die Wirkung von Vitamin E in Gegenwart von gefäßerweiternden und/oder durchblutungsfördernden Mitteln erheblich gesteigert wird, und dadurch die Behandlungszeit verkürzt wird. Die Symptome gehen schneller zurück.

Auch das Eindringen von Vitamin E durch die Haut wird überraschenderweise durch die Gegenwart von durchblutungsfördernden Mitteln, wie Heparin Natrium, Extr. Hippocastani etc., besonders erhöht und demzufolge in seiner Wirkung erheblich gesteigert. Bei der Verwendung von Heparin Natrium wird die hohe Dosierung von 30.000 bis 150.000 i.E. bevorzugt. Es wurde gefunden, daß bei diesen Wirkstoffen in Kombination mit ausreichend dosiertem Vitamin E die Behandlungsdauer wesentlich verkürzt werden kann. Die Krankheitsymptome gehen rascher zurück, so daß nach einiger Zeit auch die Dosierung reduziert werden kann.

Diese Ergebnisse waren nicht vorhersehbar und ermöglichen eine Therapie, bei der ein Teil des chemischen Wirkstoffes durch einen Naturstoff ersetzt wird, der sich obendrein praktisch in jeder Körperzelle befindet.

Mittel, die die Wirkung von Vitamin E erheblich steigern und dadurch für die Erfindung verwendet werden können, sind durchblutungsfördernde Mittel, wie sie oben im einzelnen genannt wurden.

Es wurde gefunden, daß bei Applikation dieser Mittel in Kombination mit ausreichend dosiertem Vitamin E die Symptome bei zahlreichen Patienten rascher zurückgehen und nach mehreren Monaten die Menge dieser durchblutungsfördernden Mittel verringert werden kann.

Zur Behandlung von Erkrankungen des Venen- und Analbereichs sind Mittel in Salbenform gemäß der Erfindung bevorzugt. Sie enthalten in einer weiteren bevorzugten Ausführungsform gemäß der Erfindung Vitamin E in einer Menge von 7 bis 10 Gew.-%. Die bevorzugten, als durchblutungsfördernde Verbindungen verwendeten Substanzen sind Mittel auf pflanzlicher Basis. Als Beispiel derartiger pflanzlicher Mittel kann Extractum Hippocastani genannt werden. Aber auch Heparin ist als bevorzugt zu nennen.

Als übliche Salben-, oder Cremegrundlagen können Eucerin cum aqua, Ungt. Cordes, Ungt. Emulsificans, sowie andere nicht wasserlösliche Salbengrundlagen bzw. deren Gemische verwendet werden. Geeignete Salbengrundlagen sind beispielsweise Wollwachs, Vaseline DAB 8, Paraffin dünnflüssig sowie Gemische derselben. Sie können auch Emulgatoren enthalten wie Cetylstearylalkohol etc..

Auch geeignet als Salbengrundlagen sind Unguentum alcoholum lanae aquosum mit ca. 5 bis 10% Cetiol (Ölsäureoleylester) sowie Unguentum lanette, 24 T Cetylstearylalkohol, 16 T Cetiol DAB 8, 60 T aqua conservata.

Bei dieser Kombination zieht Vitamin E sofort in die Haut ein. Zu dieser Kombination können selbstverständlich weitere Vitamine, wie z.B. Vitamin B1, B2 und B6 und verträgliche Schmerzmittel sowie Lokalanaesthetika zugesetzt werden. Lokalanaesthetika sind gefäßerweiternd. Sie können zu den Salben als Oberflächenanaesthetikum, wie Anaesthesin (Ethaform) sowie Tetracain (Pantocain), zugegeben werden.

Die vorliegende Erfindung beschreibt demzufolge Einreibungen, z.B. Creme, Gel und Salbe oder Lotion, enthaltend Vitamin E, die insbesondere folgende Bestandteile enthalten:

80 bis 30 Gew.-% Wasser, vorzugsweise
70 bis 30 Gew.-% Wasser, vorzugsweise 60 bis 40 Gew.-%
30 bis 5 Gew.-% Cetiol (Oleyloleat), vorzugsweise
25 bis 7 Gew.-%,
30 bis 2 Gew.-% Cetyl-Stearylalkohol oder andere aliphatische Alkohole, vorzugsweise 25 bis 2 Gew.-%.

Man kann anstelle von Cetyl-Stearylalkohol auch ganz oder teilweise andere emulgierende Alkohole, z.B. aliphatische Alkohole oder Wollwachsalkohol bzw. Diole, Stearinol, Monoglyceride mit aliphatischen Säuren verestert oder ähnliche Stoffe verwenden. Man kann z.B. auch Paraffin oder Vaseline zusetzen, um die Salbe streichfähig zu machen. Auch Cetiol (Oleyloleat) kann durch andere Emulgatoren, wie z.B. Tween[R] 20 oder Tween[R] 80 etc., ganz oder teilweise ersetzt werden.

Eine besonders gute Kombination als Grundlage für Vitamin-E-haltige Salben bzw. Cremes stellt die Kombination aus
30 bis 20 Gew.-% Cetyl-Stearylalkohol,
20 bis 10 Gew.-% Cetiol (oleyl oleat),
60 bis 40 Gew.-% Wasser (aqua conservata)
dar.

Diese Vitamin-E-haltige Salbe zieht sofort in die Haut ein.

Es ist bekannt, daß wasserhaltige Salbengrundlagen, wie Unguentum emulsificans aquosum und Unguentum alcoholum lanae aquosum zur Verarbeitung von wasserlöslichen Wirkstoffen geeignet sind. Hier überrascht, daß wasserhaltige Salbengrundlagen, die fast über 50% wasserhaltig sind, sehr gut zur Verarbeitung fettlöslicher Wirkstoffe wie Vitamin E, geeignet sind.

Als hautreizende Mittel bzw. Hautdurchblutungsmittel sind beispielsweise auch folgende Substanzen geeignet: Ol. Juniperi, Ol. Pini Pumilionis (Latschenkiefernöl), Ol. Eucalypti, Ol. Rosmarinae und Tinct. Camphorae bzw. Kampfer.

Als gefäßerweiterndes Pflanzenmittel ist z.B. Extract. Calendulae aus Blume und Herba calendulae zu nennen. Es wurde festgestellt, daß diese gefäßerweiternden bzw. Durchblutungsmittel die Wirkung von Vitamin E erheblich steigern, bzw. die Behandlungsdauer verkürzen und die Schmerzen auf lange Sicht beseitigen. Es können auch weitere durchblutungs- bzw. gefäßerweiternde Mittel, wie z.B. Trimethylolrutosid, verwendet werden.

Überraschenderweise wurde ferner gefunden, daß hochdosiertes Vitamin E mit durchblutungsfördernden Mitteln oder Derivate als Venenmittel besondere Vorteile bringt, wenn Vitamin A zugesetzt wird. Hierbei wird insbesondere die Behandlungsdauer verkürzt. Diese Erfindung beschreibt daher weiterhin Venenmittel, enthaltend Vitamin A und E und durchblutungsfördernde Mittel.

Vitamin A und E neigen insbesondere in Gegenwart von anderen Wirkstoffen im wäßrigen Medium sehr stark zu Klumpenbildung. Daher besteht die Gefahr, daß die fettlöslichen wertvollen Stoffe nicht absorbiert werden.

Überraschend wurde festgestellt, daß geringe Mengen Emulgator, ca. 1% ausreichen, um die Klumpenbildung zu verhindern. Die Wirkstoffe werden leichter im wäßrigen Medium dispergiert bzw. suspendiert.

Eine größere Menge Emulgator ist nicht notwendig, da meistens 1 bis 5% ausreichend sind, um die Klumpenbildung zu verhindern. Man kann auch Emulgatoren bis zu 10% oder mehr verwenden.

Es können die üblichen Emulgatoren, die in den medizinischen Präparaten verwendet werden, wie Tween[R] 20, Cremophore[R], aliphatische Alkohole etc. verwendet werden. Für diese Erfindung werden jedoch Tween[R] 80 und Cetiol bevorzugt.

Man kann als Emulgator auch Lecithin in einer Konzentration zwischen 1 und 13% verwenden. Damit wird die Resorption von der Kombination A + E, insbesondere von A begünstigt. Geringe Mengen des Emulgators aus Lecithin sind ausreichend, um die Klumpenbildung der fettlöslichen Vitamine zu verhindern und um eine optimale Resorption zu begünstigen. Auch die Verwendung von großen Mengen Lecithin, bis zu 50%, zeigt positive Wirkung. Jedoch ist zu empfehlen, ca. 1% herkömmliche Emulgatoren wie Tween[R] 80 zuzusetzen. Dadurch wird die Mischbarkeit von Lecithin mit den beiden Vitaminen begünstigt und eine Klumpenbildung verhindert.

Besonders vorteilhaft hinsichtlich der Resoption ist die Verwendung von herkömmlichen Emulgatoren wie z.B. ca. 1% Tween[R] 80 zusammen mit 1 bis 13% Lecithin. Statt Tween[R] 80 können auch als Emulgatoren Tween[R] 20, Cetiol, Ölsäureoleylester, Cremophore[R] verwendet werden. Es wird als Lecithinpräprarat das Sojalecithin bevorzugt verwendet.

Vitamin A kann als Vitamin-A-Palmitat, als auch Vitamin-A-Acetat, als auch weiterer Ester des Vitamin A, als auch $\beta$-Carotin verwendet werden.

Vitamin E kann in allen seinen alpha-Formen verwendet werden, sowohl als freies als auch als Ester. Dieser Ester kann als Acetat, Succinat oder als anderer Ester verwendet werden.

Es können auch weitere Zusätze wie Vitamine, z.B. Vitamine der B-Reihe, oder Analgetika etc. zugesetzt werden.

Die durchblutungsfördernden Mittel können ebenfalls in Retard-Form verwendet werden.

Es wurde überraschenderweise gefunden, daß die Kombination von Benzaron mit Vitamin E bzw. Vitamin A + E die nachteiligen Wirkungen beseitigt, die bei der An-wendung der bisher bekannten Benzaronsalben auftreten. Außerdem wird die Wirkung von Benzaron auf die Venen dadurch erheblich gesteigert und intensiviert. Die Behandlungsdauer verkürzt sich wesentlich im Vergleich zu der Behandlung mit Benzaron ohne den erfindungsgemäßen Zusatz von Vitamin A und E.

Bei der Herstellung von Salben und Cremes wird Vitamin E zwischen 5 und 20 Gew.-%, vorzugsweise zwischen 7 und 10 Gew.-% verwendet. Typische Beispiele liegen zwischen 3 und 10 Gew.-%. Der Gehalt an Benzaron beträgt bis zu 12 Gew.-%, vorzugsweise 3 bis 9 Gew.-%.

Es können die üblichen bekannten Salben-, Creme und Milchgrundlagen wie Ungt. cordes, Eucerin cum aqua, Polyäthylenglycol-Salbengrundlagen, Stearinol, Monoglyceride mit aliphatischen Säuren verestert und Triglyceride oder deren Gemische mit aliphatischen Alkoholen oder deren Derivate, wie emulgierender Cetyl--Stearylalkohol sowie Wollwachsalkohol oder Diole, verwendet werden.

Man kann auch Paraffin oder Vaseline[R] oder andere geeignete Stoffe zusetzen, um die Salbe streichfähig zu machen. Auch Cetiol (Oleyloleat) kann durch andere Emulgatoren, z.B. Tween[R] 20 oder 80 etc., ganz oder teilweise ersetzt werden.

Es wurde jedoch gefunden, daß die beste Kombination als Grundlage für Vitamin-E-haltige Salben bzw. Cremes folgende ist:

30 bis 20 Gew.-% Cetyl-Stearylalkohol
20 bis 10 Gew.-% Cetiol (oleyl oleat)
60 bis 40 Gew.-% Wasser (aqua conservata)

Diese Vitamin-E-haltige Salbe zieht sofort in die Haut ein.

Weitere nützliche bekannte Zusatzstoffe, wie Vitamin A, ein oder mehrere Vitamine der B-Reihe, sowie Lebertran, auch ungesättigte Fettsäuren, wie z.B. Linolsäure, Octadeca-9,11-diensäure oder Siliconöl bzw. Polysiloxsäure, können zu den Vitamin-E-Einreibungen zugesetzt werden, um bessere Eigenschaften zu erzielen.

Maßgebend für diese Erfindung ist der Beweis, daß Vitamin E besonders in seinen freien Formen, z.B. DL-alpha-Tocopherol und/oder D-alpha-Tocopherol, transdermal resorbierbar ist. Vitamin A kann in Form von Acetat oder Palmitat oder irgendeiner anderen geeigneten Form z.B $\beta$-Carotin zugesetzt werden. Benzaron oder dessen Derivate bzw. Gemische können verwendet werden.

Es wurde jetzt gefunden, daß Vitamin E überraschenderweise auch hervorragend zur Behandlung von Erkrankungen des Analbereiches geeignet ist. Insbesondere eignet es sich zur Behandlung von Hämorrhoiden, Varizen, altersbedingter Venenschwäche, Kapillaropathie, Thrombophlebitis im Analbereich, Pruritus ani, Analfissuren, Analrhagaden und feuchten Analexzemen.

Dieser neue Indikationsbereich war aufgrund des bisherigen Wissensstandes nicht vorherzusehen und eröffnet ein neues und breites Anwendungsfeld für Vitamin E.

Für die neue Verwendung des Vitamin E zur Behandlung von Erkrankungen des Analbereiches eignen sich in besonderem Maße Salben.

Diese neue Applikationsform des Vitamin E ist aus zwei Gründen besonders bevorzugt: Mit ihrer Hilfe ist es möglich, das Vitamin E verzögerungsfrei und in relativ hohen Konzentrationen in den gewünschten Bereich zur Verfügung zu stellen. Weiterhin werden die Verluste an Vitamin E bei oraler Applikation vermieden, da bekannt ist, daß Vitamin E in erheblichem Maße durch Magensäure zerstört wird.

Die erfindungsgemäßen Salben enthalten Vitamin E und ein oder mehrere der oben genannten durchblutungsfördernden bzw. gefäßerweiternden Mittel und werden auf Basis üblicher Salbenbasen hergestellt. Sie können gewünschtenfalls weitere übliche und bekannte Wirkstoffe zur Behandlung von Erkrankungen des Analbereiches enthalten. Zu den gefäßerweiternden und/oder durchblutungsfördernden Mitteln zählen insbesondere Heparin Natrium, Extract. Hippocastani, Extract. bzw. Tinct. arnicae, $\beta$-Hydroxyäthylrutoside, Salicylsäureester, Nikotin-säu-reester, insbesondere der Nikotinsäure-benzylester und Flunarizindihydrochlorid.

Als übliche Salben-, oder Cremegrundlagen können Eucerin cum aqua, Ungt. Cordes Ungt. emulsificans, sowie andere nicht wasserlösliche Salbengrundlagen bzw. deren Gemische verwendet werden. Geeignete Salbengrundlagen sind beispielsweise Wollwachs, Vaseline DAB 8, Paraffin dünnflüssig sowie Gemische derselben. Sie können auch Emulgatoren enthalten wie Cetylstearylalkohol etc.

Zur Behandlung der Erkrankung des Analbereiches wird Vitamin E anfangs mehrmals täglich, später nur noch einmal täglich angewendet.

Salben enthalten 5 bis 20 Gew.-% Vitamin E. Als Vitamin E kommt sowohl natürliches D-alpha-Tocopherol und seine Konzentrate oder synthetisches D,L-alpha-Tocopherol in Frage. Die für orale Zubereitungen in üblicher Weise verwendeten Acetate kommen für Salben nicht in Frage, da die Acetate in ungespaltener Form unwirksam sind und bei der Resorption im Analbereich nicht in das freie Vitamin E gespalten werden.

In den nachfolgenden Beispielen werden Venenmittel näher erläutert:

## Beispiel 1

100 g Salbe enthalten:
400 mg Allantoin;
400 mg Dexapanthenol;
5000 mg D-alpha-Tocopherol;
30000 I.E. Heparin Natrium;

## Beispiel 2

100 g Salbe enthalten:
2,5 g O-($\beta$-Hydroxyäthyl)-Rutoside;
7,5 g D-alpha-Tocopherol oder D,L-alpha-Tocopherol;

## Beispiel 3

100 g Salbe enthalten:

400 mg Allantoin;
400 mg Dexapanthenol;
8,8 g D-alpha-Tocopherol oder D,L-alpha-Tocopherol;
30000 I.E. Heparin Natrium

**Beispiel 4**

100 g Salbe enthalten:
4,5 g Extract Hippocastani (enthält ca. 800 mg Aescin);
8,0 g D-alpha-Tocopherol;

**Beispiel 5**

100 g Gel enthalten:
50000 I.E. Heparin Natrium;
12 g Arnikablüten-Extract ((1:10) Alkohol 60%);
25 g Tinct. Hippocastani e sem. 1:1 entspricht 0,65 Aescin;
7,5 g D-alpha-Tocopherol;

**Beispiel 6**

100 g Gel enthalten:
7,0 g $\beta$-Hydroxyäthyl-Salicylat;
7,0 g D-alpha-Tocopherol;

**Beispiel 7**

Rheumapflaster 15 x 25 cm enthält bzw. ist einseitig präpariert mit:
70 mg Extract Arnicae;
70 mg Extract Capsici;
30 mg Extract Bella donae;
1500 mg D-alpha-Tocopherol-Konzentrat;

**Beispiel 8**

100 g Salbe enthalten:
10 g Benzocain (Anaesthesin);
8 g D-alpha-Tocopherol-Konzentrat;
1 g Benzylnicotinat;

**Beispiel 9**

100 g Salbe enthalten:
3 g Hydroxyäthyl-Salicylat;
1 g Benzylnicotinat;
7 g D-alpha-Tocopherol;

**Beispiel 10**

100 g Salbe enthalten:
8 g D-alpha-Tocopherol;
400 mg Allantoin;
400 mg Dexapanthenol;
150000 I.E. Heparin Natrium;

**Beispiel 11**

Tropfen enthalten:

100 ml 90% Äthylalkohol;
40 g D,L-alpha-Tocopherolacetat;
4,5 g Extract Hippocastani (enthalten 750 mg Aescin);

**Beispiel 12**

Eine Salbe enthält
10 g D-alpha-Tocopherol
50.000 I.E. Heparin Natrium
ad 100 Salbengrundlage aus
22 T Cetyl-Stearylalkohol
18 T Cetiol
60 T Wasser

**Beispiel 13**

7 g Vitamin E (D-alpha-Tocopherol)
1 g Nicotinsäurebenzylester
1 g Campher
ad 100 Salbengrundlage aus
17 T Cetyl-Stearylalkohol
8 T Weiße Vaseline
15 T Cetiol
60 T Wasser (aqua conservata)

**Beispiel 14**

7 g Vitamin E
15 g Tinct. calendualae
ad 100 Salbengrundlage aus
13 T Wollwachsalkohol
2 T Cetyl-Stearylalkohol
20 T Cetiol
5 T Paraffin
50 T Wasser (aqua conservata)

**Beispiel 15**

8 g Vitamin E (DL-alpha-Tocopherol)
1,5 g Rosmarinöl
1 g Extract Hippocastani (standarisiert auf mind. 8% Aescin)
1 g Öl juniperi
ad 100 Salbengrundlage wie Beispiel 12

**Beispiel 16**

Lösung aus
8 g Vitamin E (D-alpha-Tocopherol-Konzentrat)
1 g Latschenkiefernöl (Öl pini Pumilionis)
1 g Eucalyptusöl
1 g Öl juniperi
ad 100 Isopropylalkohol

**Beispiel 17**

7 g D-alpha-Tocopherol-Konzentrat
2 g Tinct. arnicae
2 g Salicyl-$\beta$-Hydroxyäthylester

ad 100 Salbengrundlage wie Beispiel 12

**Beispiel 18**

Lösung gemäß Beispiel 16
7,0 g Vitamin E
1,0 g Latschenkiefernöl
1,0 g Arnikatinktur
ad 100 Isopropylalkohol

**Beispiel 19**

9,0 g Vitamin E
20,0 g Tinct. Calendulae
ad 100 Salbengrundlage wie 12

**Beispiel 20**

```
100 ml Tropfen aus Äthylalkohol enthalten
Cinnarizin                          7,5 g
Vitamin E                           4,0 g
Vitamin A Palmitat          2,5 Millionen Ein-heiten
```

Die folgenden Beispiele betreffen verstärkte Venenmittel enthaltend Benzaron und Vitamin E.

**Beispiel 21**

9 g D-alpha-Tocopherol-Konzentrat
2 g Benzaron
ad 100,0 aus einer Salbengrundlage, bestehend aus 20 Gew.-% Cetylstearylalkohol, 20 Gew.-% Cetiol und 60 Gew.-% aqua conservata.

**Beispiel 22**

10 g DL-alpha-Tocopherolacetat
2 g Benzaron
ad 100 aus einer Salbengrundlage wie unter 21, jedoch anstelle von 60 Gew.-% Wasser nur 55 Gew.-% und 5 Gew.-% Vaselinum Album.

**Beispiel 23**

25.000 I.E. Vitamin-A-Palmitat
400 mg dl-alpha-Tocopherolacetat
200 mg Benzaron
100 mg Sojabohnenöl
In den nachfolgenden Beispielen ist die Herstellung von Salben zur Behandlung von Erkrankungen des Analbereiches in einigen typischen Zusammensetzungen beschrieben. Prinzipiell können aber auch andere Rezepturen zur Anwendung kommen.

**Beispiel 24**

Wollwachs DAB 8 (30 Gew.-%), Vaseline DAB 8 (20 Gew.-%) und Paraffin dünnflüssig (50 Gew.-%) werden miteinander vermischt und mit D-alpha-Tocopherol oder D,L-alpha-Tocopherol zu einer Salbe verrührt, so daß 7,5 g Vitamin E in 100 g Salbe enthalten sind.

Weitere Salbenrezepturen wurden hergestellt mit D,L-alpha-Tocopherol mit 6,5 Gew.-% Ext. Sem. Hippocast. (Spir. Spiss 5:1), 12,0 Gew.-% basischen Bismutgallat und 0,15 Gew.-% Dexapanthenol.

Eine weitere Salbe enthielt 10.000 I.E. Heparin Natrium und 8 Gew.-% D-alpha-Tocopherol.

Eine ähnliche Rezeptur enthielt 10.000 I.E. Heparin Natrium und 7,5 Gew.-% D-alpha-Tocopherol oder D,L-alpha-Tocopherol.

Weitere Salbenrezepturen enthielten in 100 g 0,25 g Rosskastanien-Extrakt (standardisiert auf mindestens 8% Aescin), Hamamelis-Extrakt (alkoholwässriger-Extrakt) 1,5 g, 12 g Zinkoxid und 7,5 g D-alpha-Tocopherol-Konzentrat, oder

2,5 g O-($\beta$-Hydroxyäthyl)-rutoside und 7,5 g D-alpha-Tocopherol-Konzentrat oder D,L-alpha-Tocopherol, oder

4,5 g Extract. Hippocastani (enthaltend ca. 800 mg Aescin) und 8,0 g D-alpha-Tocopherol-Konzentrat, oder

5.000 I.E. Heparin Natrium, 12 g Arnikablütenextrakt (1:10 Alkohol 60%), 25 g Tinct. Hippocastani e sem. 1:1 entsprechend 0,65 Aescin und 7,5 g D-alpha-Tocopherol, oder

7,0 g $\beta$-Hydroxyäthyl-Salicylat und 7,0 g D-alpha-Tocopherol.

**Beispiele 25 - 27**

25) Eine Analsalbe enthält
210 I.E. Heparin Natrium
8,0 g D-alpha-Tocopherol Konzentrat
ad 100 Salbengrundlage aus
22 g Cetyl-Stearylalkohol,
18 g Cetiol und
60 g Wasser.

26) 7,0 g Vitamin E DL-alpha-Tocopherol 6,0 g Kondensationsprodukte aus Metakresol (zu 95% Monosulfonsäure) und Formaldehyd mittleres Molekulargewicht 350 bis 600)
1,0 g Benzocain
ad 100 Salbengrundlage aus
17,0 g Cetyl-Stearylalkohol
8,0 g weiße Vaseline
10,0 g Paraffin
15,0 g Cetiol
50,0 g Aqua conservata

27) Eine Salbe enthält 150 mg wässrige Suspension die korpuskularen Bestandteile und Stoffwechselprodukte von Escherichia coli ca. 300 Mio
3 mg Phenol liquifactum
80 mg Vitamin E
ad 1,0 g Salbengrundlage wie 2)

**Ansprüche**

Die Beispiele 28 und 29 betreffen Venenmittel, die Vitamin A und E sowie Lecithin enthalten.

```
29) Nicotinsäure                    300 mg
    Vitamin E                       400 mg
    Vitamin-A-Palmitat              50.000 I.E.
    Cetiol (Oleylsäureester)        10 mg
    Sojaöl                          100 mg
    Sojalecithin                    20 mg
```

```
28) 100 ml Tropfen aus Äthylalkohol enthalten
    Cinnarizin                      7,5 g
    Vitamin E                       4,0 g
    Vitamin-A-Palmitat              2,5 Millionen
                                    Einheiten
    Lecithin                        2,5 g
```

1. Mittel zur Behandlung von Erkrankungen der Venen und des Analbereichs im Wege der transdermalen Resorption der Wirkstoffe, gekennzeichnet durch einen Gehalt an Vitamin E im Bereich von 5 bis 20 Gew.-%, an einem oder mehreren gefäßerweiternden und/oder durchblutungsfördernden Mitteln aus der Gruppe Heparin, o-(β-Hydroxyethyl-)Rutoside, Trimethylolrutosid, Extractum Hippocastani, Extractum Arnicae, Extractum Calendulae, Ol. Juniperi, Ol. Pini Pumilionis, Ol. Eucalypti, Ol. Rosmarinae, Tinct. Camphorae, Campher, Salicylsäure und Salicylate, β-Hydroxyethylsalicylat, Nicotinsäure und Nicotinate, Xantinolnicotinat, Inositolnicotinat, Cinnarizin, Bencyclanhydrogenfumarat, Dihydroergotoxinmethansulfonat, Dihydroergocorninmethansulfonat, Dihydroergocristinmethansulfonat, Pentoxifyllin, β-Pyridylcarbinol, Bamethansulfat, Ginkgoflavonglykoside, Flunarizin und dessen Hydrochlorid und Buflomedil, sowie gegebenenfalls an weiteren Wirkstoffen und üblichen Träger- und Hilfsstoffen.

2. Mittel nach Anspruch 1 in Salbenform.

3. Mittel nach einem der Ansprüche 1 oder 2, gekennzeichnet durch einen Gehalt an Vitamin E im Bereich von 7 bis 10 Gew.-%.

4. Mittel nach einem der Ansprüche 1 bis 3, dadurch gekennzeichnet, daß sie pflanzliche durchblutungsfördernde Mittel enthalten.

5. Mittel nach einem der Ansprüche 1 bis 4, dadurch gekennzeichnet, daß sie zusätzlich Emulgatoren enthalten.

6. Mittel nach einem der Ansprüche 1 bis 5, dadurch gekennzeichnet, daß sie zusätzlich Kondensationsprodukte aus sulfonierten Kresolen (zu ca. 95 % Monosulfonsäuren enthaltend) und Formaldehyd enthalten.

7. Mittel nach einem der Ansprüche 1 bis 6, dadurch gekennzeichnet, daß die zusätzlich eine wäßrige Suspension von korpuskulären Bestandteilen und Stoffwechselprodukten von Escherichia coli enthalten.

8. Mittel nach einem der Ansprüche 1 bis 7, dadurch gekennzeichnet, daß sie Vitamin E natürlichen oder synthetischen Ursprungs in Form von D-alpha-Tocopherol, D,L-alpha-Tocopherol, und/oder Estern enthalten.

9. Mittel nach Anspruch 8, dadurch gekennzeichnet, daß die Ester Acetate und/oder Succinate sind.

## Claims

1. Agents for treating diseases of the veins and of the anal region by the route of transdermal resorption of the active ingredients, characterized by a content of vitamin E within the range of from 5 to 20% by weight, of one or more vasodilative and/or blood circulation-promoting agents from the group consisting of heparin, o-(β-hydroxyethyl)-rutoside, trimethylolrutoside, Extractum Hippocastani, Extractum Arnicae, Extractum Calendulae, Ol. Juniperi, Ol. Pini Pumilionis, Ol. Eucalypti, Ol. Rosmarinae, Tinct. Camphorae, camphor, salicylic acid and salicylates, β-hydroxyethyl salicylate, nicotinic acid and nicotinates, xanthinol nicotinate, inositol nicotinate, Cinnarizine, Bencyclane hydrogenfumarate, dihydroergotoxine methanesulfonate, dihydroergocornine methanesulfonate, dihydroergocristine methanesulfonate, Pentoxifylline, β-pyridylcarbinol, Bamethan sulfate, Gingko flavoglycosides, Flunarizine and its hydrochloride and Buflomedil, and optionally of further active substances and conventional carrier and auxiliary materials.

2. The agents according to claim 1 in the form of an ointment.

3. The agents according to either of claims 1 or 2, characterized by a content of vitamin E within the range of from 7 to 10% by weight.

4. The agents according to any one of claims 1 to 3, characterized in that they contain vegetable blood circulation-promoting agents.

5. The agents according to any one of claims 1 to 4, characterized in that they additionally contain emulsifiers.

6. The agents according to any one of claims 1 to 5, characterized in that they additionally contain condensation products of sulfonated cresols (containing about 95% of monosulfonic acids) and formaldehyde.

7. The agents according to any one of claims 1 to 6, characterized in that they additionally contain an aqueous suspension of corpuscular components and metabolites of Escherichia coli.

8. The agents according to any one of claims 1 to 7, characterized in that they contain vitamin E of natural or synthetic origin in the form of D-α-tocopherol, D,L-α-tocopherol and/or esters.

9. The agents according to claim 8, characterized in that the esters are acetates and/or succinates.

## Revendications

1. Produits de traitement des maladies des veines et de la région anale par résorption transdermique de la substance active, caractérisés en ce qu'ils contiennent de la vitamine E à raison de 5 à 20 % en poids, une ou plusieurs substances dilatant les vaisseaux et/ou accélérant la circulation du sang, choisies dans le groupe comprenant les substances suivantes : héparine, o-(β-hydroxyéthyl-)rutoside, triméthylolrutoside, extractum hippocastani, extractum arnicae, extractum calendulae, Ol. juniperi, Ol. pini pumilionis, Ol. eucalypti, Ol. rosmarinae, tinct. camphorae, camphre, acide salicylique et salicylate, salicylate de β-hydroxyéthyle, acide nicotinique et nicotinates, nicotinate de xanthinol, nicotinate d'inositol, cinnarizine, fumarate acide de bencyclane, méthane-sulfonate de dihydro-ergotoxine, méthane-sulfonate de dihydro-ergocarnine, méthane-sulfonate de dihydro-ergocristine, pentoxifylline, β-pyridylcarbinol, sulfate de baméthane, glucoside de gingkoflavone, flunarizine et son chlorhydrate, et buflomedile, ainsi qu'éventuellement d'autres substances actives, et des véhicules et adjuvants usuels.

2. Produits selon la revendication 1, sous forme de pâte.

3. Produits selon l'une des revendications 1 ou 2, caractérisés par une teneur en vitamine E de 7 à 10 % en poids.

4. Produits selon l'une des revendications 1 à 3, caractérisés en ce qu'ils contiennent des substances d'origine végétale accélérant la circulation du sang.

5. Produits selon l'une des revendications 1 à 4, caractérisés en ce qu'ils contiennent en outre des émulsifiants.

6. Produits selon l'une des revendications 1 à 5, caractérisés en ce qu'ils contiennent en outre des produits de condensation de crésols sulfonés (contenant jusqu'à environ 95 % d'acides mono-sulfonés) et du formaldéhyde.

7. Produits selon l'une des revendications 1 à 6, caractérisés en ce qu'ils contiennent en outre une suspension aqueuse de composants corpusculaires et de produits de métabolisme de Escherichia coli.

8. Produits selon l'une des revendications 1 à 7, caractérisés en ce qu'ils contiennent des sources naturelles ou synthétiques de vitamine E sous forme de D-alpha-tocophérol, D,L-alpha-tocophérol, et/ou d'esters.

9. Produits selon la revendication 8, caractérisés en ce que les esters sont des acétates ou des succinates.